(19) Europäisches Patentamt
European Patent Office
Office européen des brevets

(11) Veröffentlichungsnummer: **0 184 057**
**B1**

(12) # EUROPÄISCHE PATENTSCHRIFT

(45) Veröffentlichungstag der Patentschrift: **11.04.90**

(51) Int. Cl.⁵: **G 03 C 7/34** // C07C309/86

(21) Anmeldenummer: **85114565.6**

(22) Anmeldetag: **16.11.85**

(54) **Fotografisches farbkupplerhaltiges Material.**

(30) Priorität: **30.11.84 DE 3443700**

(43) Veröffentlichungstag der Anmeldung:
**11.06.86 Patentblatt 86/24**

(45) Bekanntmachung des Hinweises auf die Patenterteilung:
**11.04.90 Patentblatt 90/15**

(84) Benannte Vertragsstaaten:
**BE DE FR GB**

(56) Entgegenhaltungen:
**EP-A-0 105 991**

**PATENT ABSTRACTS OF JAPAN, Band 9, Nr. 259 (P-397)1982r, 17. Oktober 1985; & JP-A-60 107 650 (KONISHIROKU SHASHIN KOGYO K.K.) 13-06-1985**

(73) Patentinhaber: **Agfa-Gevaert AG**
**D-5090 Leverkusen 1 (DE)**

(72) Erfinder: **Wolff, Erich, Dr.**
**Balkhauser Weg 6**
**D-5650 Solingen (DE)**
Erfinder: **Langen, Hans, Dr.**
**Weidengarten 16**
**D-5300 Bonn (DE)**
Erfinder: **Braden, Rudolf, Dr.**
**Nothauser Feld 1**
**D-5068 Odenthal (DE)**

**EP 0 184 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft ein farbfotografisches Material mit neuen einemulgierten phenolischen Blaugrünkupplern mit Phenylureidostruktur.

Es ist bekannt, farbige fotografische Bilder durch chromogene Entwicklung herzustellen, d.h. dadurch, daß man bildmäßig belichtete Silberhalogenidemulsionsschichten in Gegenwart geeigneter Farbkuppler mittels geeigneter farbbildender Entwicklersubstanzen — sogenannter Farbentwickler — entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbentwickler werden gewöhnlich aromatische, primäre Aminogruppen enthaltende Verbindungen, insbesondere solche vom p-Phenylendiamintyp, verwendet.

An die Farbkuppler, sowie an die daraus durch chromogene Entwicklung erhaltenen Farbstoffe werden in der Praxis eine Reihe von Forderungen gestellt. So soll die Kupplungsgeschwindigkeit der Farbkuppler mit dem Oxidationsprodukt des Farbentwicklers möglichst groß sein. Die Farbkuppler sowie die daraus erhaltenen Farbstoffe müssen hinreichend stabil sein gegenüber Licht, erhöhter Temperatur und Feuchtigkeit. Dies gilt sowohl für frisches Material, als auch für verarbeitetes Material. Beispielsweise darf der in den Bildweißen des verarbeiteten Materials noch vorhandene restliche Kuppler nicht vergilben. Außerdem sollen die Farbstoffe hinreichend beständig sein gegenüber gasförmigen reduzierenden oder oxidierenden Agentien. Sie müssen ferner diffusionsfest in der Bildschicht verankert sein und sollen sich bei der chromogenen Entwicklung als möglichst feines Korn abscheiden. Die mechanischen Eigenschaften der Schichten dürfen durch die Farbkuppler nicht beeinträchtigt werden. Schließlich müssen die aus den Farbkupplern bei der chromogenen Entwicklung entstehenden Farbstoffe eine günstige Absorptionskurve aufweisen mit einem Maximum, das der Farbe des jeweils gewünschten Teilbildes entspricht, und möglichst geringen Nebenabsorptionen. So soll ein Blaugrünfarbstoff im Idealfall rotes Licht nahezu vollständig absorbieren und grünes sowie blaues Licht weitgehend durchlassen. Außerdem sollen die Absorptionsmaxima der Farbstoffe sowohl bei Colorumkehr- wie auch bei Colornegativfilmen möglichst mit dem Sensibilisierungsmaxima der zum Kopieren verwendeten Colorpapiermaterialen übereinstimmen.

Als Blaugrünkuppler, d.h. als Farbkuppler, die zur Erzeugung des blaugrünen Teilbildes geeignet sind, werden im allgemeinen Verbindungen verwendet, die sich von Phenol oder α-Naphthol ableiten. Naphtholische Blaugrünkuppler weisen ein sehr schlechtes Darkfadingverhalten auf, d.h. das bei der Entwicklung gebildete Blaugrün-Teilbild bleicht bei Langzeitlagerung bzw. bei einem verkürzten Test bei erhöhter Temperatur in verstärktem Maße aus, woraus eine Verrötlichung der Kopie oder ein dichteabhängiger Farbstich resultiert.

Aus US—A—2 772 162 und US—A—2 369 929 geht hervor, daß sich diesbezüglich Blaugrünkuppler mit einer 2,5-Diacylaminophenolstruktur wesentlich günstiger verhalten. Allerdings haben solche Kuppler wiederum den Nachteil, daß die daraus gebildeten blaugrünen Farbstoffe im Vergleich zu den obengenannten naphtholischen Kupplern um 20 bis 50 nm kürzerwellig absorbieren und demzufolge nicht zu einer üblichen langwelligen Rotsensibilisierung des Kopierpapieres passen.

DE—B—1 163 144 lehrt nun, daß durch Einführung eines Ureidorestes in der 2-Stellung des aromatischen Systems Kuppler resultieren, die beständige Azomethinfarbstoffe liefern.

Aus GB—A—1 111 554 geht hervor, daß die kurzwellige Absorption durch geeignete Substitution im Phenylureidoteil in den längerwelligen Bereich verschoben werden kann.

Kuppler gemäß EP—A—28 099 mit einem p-Cyano-Substituenten sowie Kuppler nach EP—A—67 689, 73 145, 73 146 und 84 100 absorbieren, bezogen auf die Azomethinfarbstoffe der derzeitig eingesetzten naphtholischen Kuppler, um 10 bis 20 nm zu kurzwellig, was aus den obengenannten Gründen für den negativ-positiv-arbeitenden Kopierprozeß noch nicht ideal ist. Auch läßt die Konstanz des Absorptionsmaximums des aus dem Kuppler entwickelten Farbstoffs in Abhängigkeit von der Dichte erheblich zu wünschen übrig. Um diesen Nachteil zu beheben, wird in der EP—A—116 428 die Kombination von phenolischen Blaugrünkupplern und nicht-farbkuppelnden diffusionsfesten Phenolverbindungen beschrieben. Aber auch diese Kombination kann die oben beschriebene Abhängigkeit von $\lambda_{max}$ und Dichte nicht befriedigend beseitigen.

Weiterhin sind die Kupplungsgeschwindigkeiten der bekannten Kuppler relativ gering. Dagegen ist wünschenswert, zur Erzielung eines feinen Farbkorns besonders in den hochempfindlichen Teilschichten schnelle Kuppler einzusetzen. Ein weiterer Nachteil der bekannten Kuppler ist ein zusätzliches Vergilben im nicht gekuppelten Bildteil.

Die Aufgabe der vorliegenden Erfindung bestand darin, Blaugrünkuppler zu finden, welche eine gute Darkfadingstabilität des gekuppelten Farbstoffs haben, insbesondere einen möglichst geringen Anstieg des Gelbschleiers, weiterhin ein genügend langwelliges Absorptionsmaximum, das dichteunabhängig ist, sowie geringe Nebendichten ergeben und welche schließlich eine hohe Kupplungsaktivität haben und in hochsiedenden Lösungsmitteln gut löslich sind.

Die Aufgabe würde gelöst mit einem lichtempfindlichen farbfotografischen Aufzeichnungsmaterial mit mindestens einer Silberhalogenidemulsionsschicht und einem dieser zugeordneten nicht-diffundierenden phenolischen Blaugrünkuppler mit einer 2-Phenylureidogruppe, dadurch gekennzeichnet, daß der Blaugrünkuppler die folgende Formel I hat.

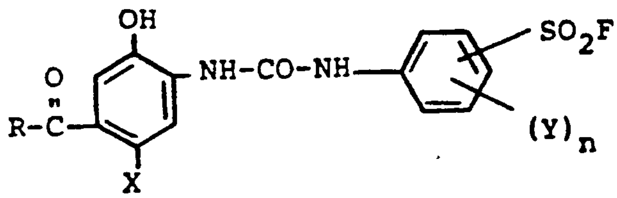

(I)

worin bedeuten:

X = H oder abspaltbare Fluchtgruppe, beispielsweise Cl, $—OCH_2CH_2SO_2CH_3$ oder $—OCH_2CONHCH_2CH_2OCH_3$,

Y = Halogen, beispielsweise Cl, Br oder F, Alkyl, beispielsweise Methyl oder Trifluormethyl, Alkoxy, beispielsweise Methoxy oder Ethoxy, Aryl oder Alkylthio,

n = 0—2, (falls n = 2, können die Reste Y gleich oder verschieden sein),

R = Ballastgruppe mit der bevorzugten Strukturformel II

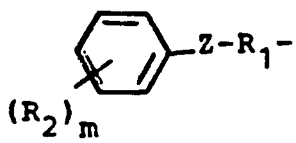

(II)

worin bedeuten:

Z = O oder S

$R_1$ = eine Alkylidengruppe mit 2 bis 20 C-Atomen der Formel $R_3$—C-Alkyl, wobei $R_3$ H oder Alkyl bedeutet.

$R_2$ = Hydroxy, Carboxy, Alkyl, Cycloalkyl, Aryl, Aralkyl, Alkoxy, Aryloxy, Alkylsulfamoyl, Arylsulfamoyl, Alkylsulfonamido, Arylsulfonamido, Alkylsulfonyl, Arylsulfonyl, Alkoxycarbonyl oder Acyloxy, worin Alkyl 1 bis 20 C-Atome enthält, worin Aryl 6 bis 20 C-Atome enthält und worin Alkyl, Aryl und Aralkyl auch beliebig mit Hydroxy, Carboxy, Alkoxycarbonyl oder Acyloxy substituiert sein können,

m = 1—3

R kann auch ein Segment aus dem Gerüst eines Polymers sein, das entstanden ist durch Polymerisation eines monomeren Kupplers der Formel

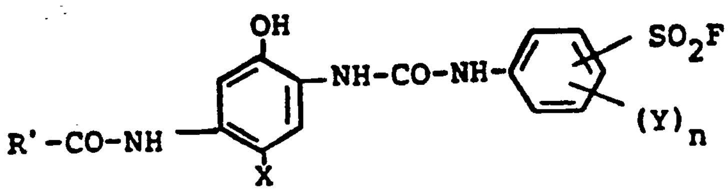

worin R' eine polymerisierbare ethylenisch ungesättigte Gruppe enthält.

Beispiele für Blaugrünkuppler gemäß der Erfindung sind nachfolgend aufgeführt.

Kuppler-Nr.

1.

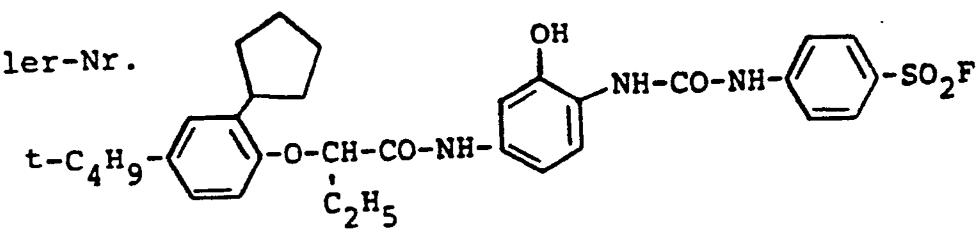

2.

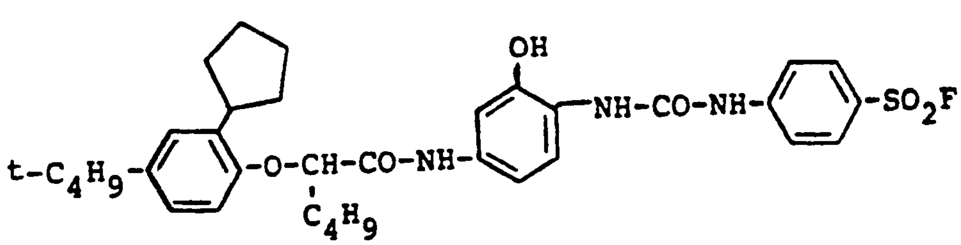

3. 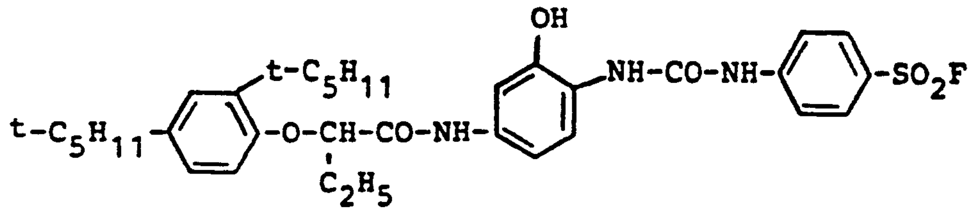

4. 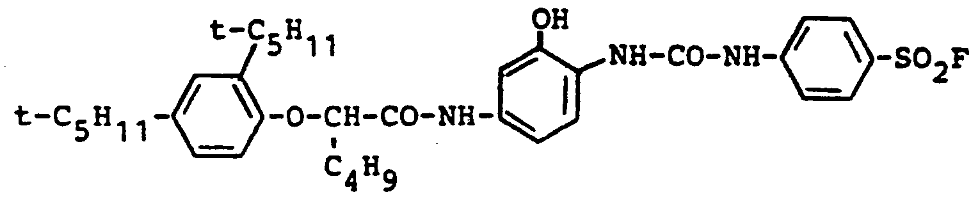

Kuppler-Nr.

5. 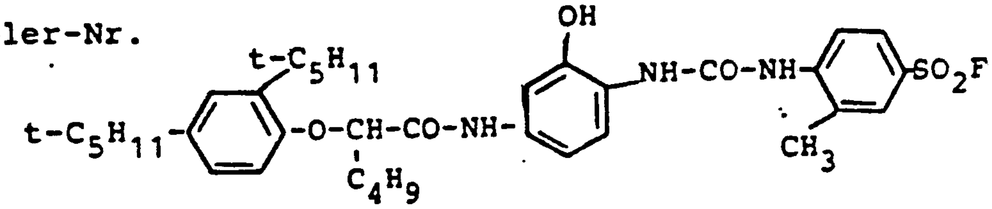

6. 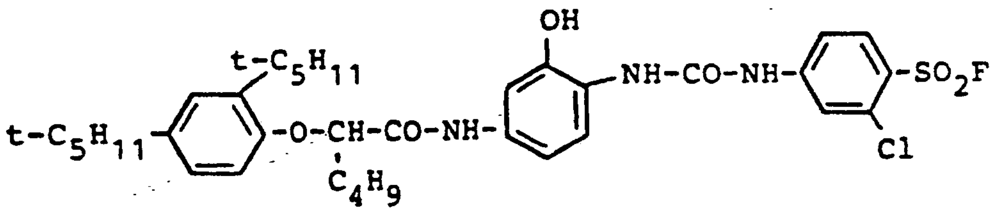

7. 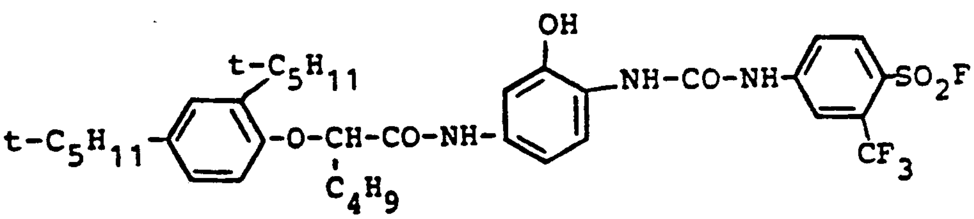

8. 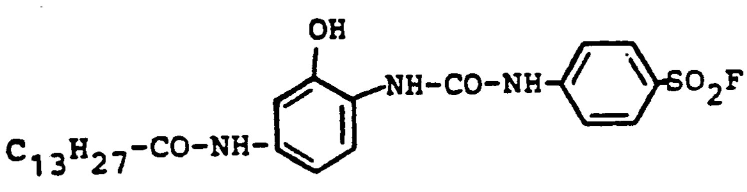

9. 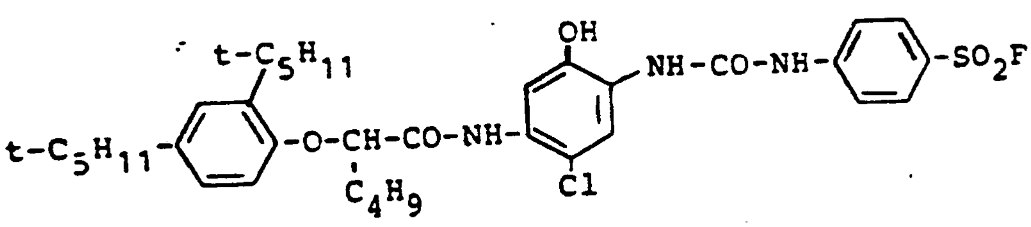

10. 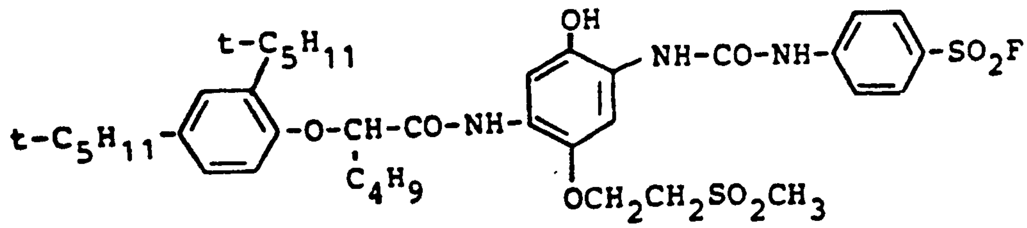

Kuppler Nr.

11. 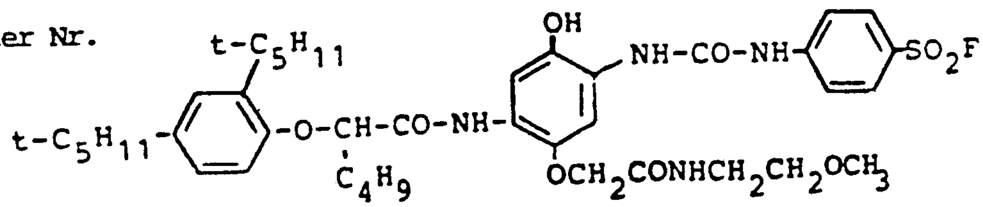

Die erfindungsgemäßen Kuppler können auf folgenden Wegen synthetisiert werden.

a) 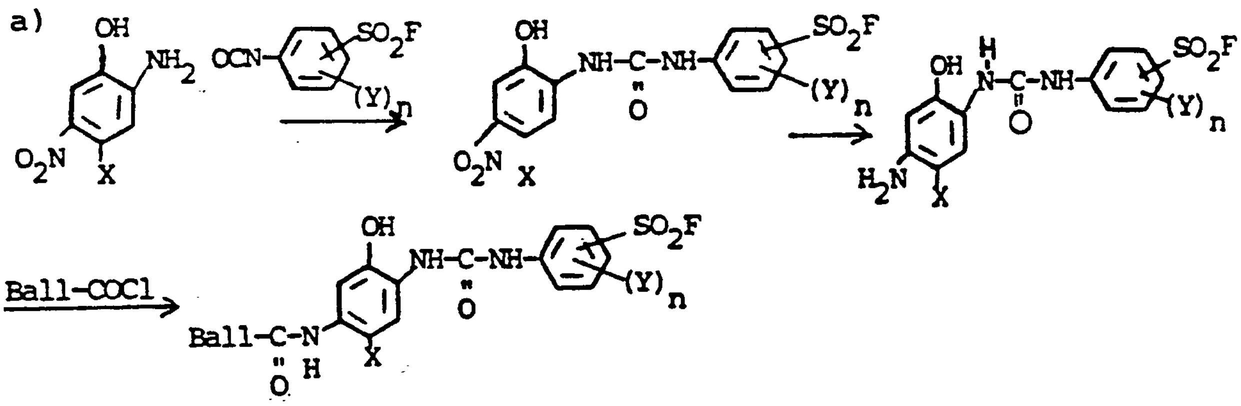


b) 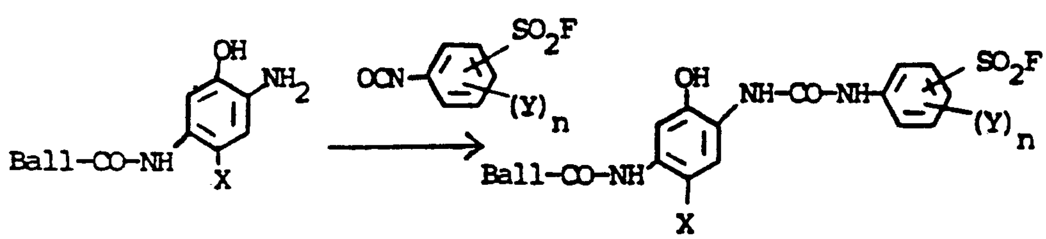

Hierin bedeutet Ball einen Ballastrest.

Die Herstellung der Kuppler, gemäß der Erfindung, ist im Nachfolgenden für Verbindung 4 auf dem Wege b) beschrieben.

Herstellung des Kupplers 4

35 g 2-Amino-5- (2',4'-di-tert-pentyl-phenoxy)hexanoylamino-phenolhydrochlorid werden in
250 ml Ether gelöst, mit
10 g Kaliumcarbonat und
15 g 4-Isocyanato-phenylsulfonsäurefluorid versetzt.

Es wird 1 Stunde bei Raumtemperatur nachgerührt, anschließend in Eis/Wasser gefällt und der abgesaugte und getrocknete Niederschlag aus Ether/Petrolether imkristallisiert.

Ausbeute: 20 g
Schmelzpunkt: 160°C
Titration mit Tetrabutylammoniumhydroxid in Dimethylsulfoxid ergibt einen Gehalt von 99,8%.

In ganz entsprechender Weise wurden die erfindungsgemäßen Kuppler 1, 3, 5 und 8 hergestellt.

Die erfindungsgemäßen Verbindungen stellen wertvolle Farbkuppler dar, die bei der chromogenen Entwicklung blaugrüne Farbstoffe mit ausgezeichneten Stabilitätseigenschaften ergeben. Sie eignen sich in hervorragender Weise für die Verwendung in lichtempfindlichen Silberhalogenidemulsionsschichten farbfotografischer Ein- oder Mehrschichtenmaterialien. Die Blaugrünkuppler müssen jedoch nicht unbedingt den lichtempfindlichen Schichten einverleibt sein; es ist vielmehr auch möglich, sie in einer

Bindemittelschicht unterzubringen, die einer lichtempfindlichen Silberhalogenidemulsionsschicht benachbart ist.

Die erfindungsgemäßen Blaugrünkuppler können nach einer der bekannten Methoden der Silberhalogenidemulsion oder aber auch einem anderen Bindemittelgemisch einverleibt werden. Da es sich bei den erfindungsgemäßen Kupplern um sogenannte Emulgierkuppler handelt, d.h. um hydrophobe Verbindungen, geschieht die Einarbeitung in bekannter Weise durch Lösen in geeigneten organischen Lösungsmitteln, z.B. in Estern, aliphatischen Carbonsäuren, insbesondere in Essigester oder Methylenchlorid, und Einemulgieren dieser Lösung in die gießfertige Silberhalogenidemulsion. Diese Methode kann gegebenenfalls durch die gleichzeitige Verwendung von öligen Kupplerlösungsmitteln modifiziert werden. Dieses Verfahren ist aus den amerikanischen Patentschriften 2 304 940 und 2 322 027 bekannt. Die erfindungsgemäßen Kuppler zeichnen sich nämlich durch eine gute Löslichkeit in hochsiedenden Lösungsmitteln wie Trikresylphosphat, Dibutylphthalat, Diethyllauramid etc. aus. Dies hat eine gute Lagerstabilität und Digestionsstabilität der für den Beguß verwendeten Emulgate infolge geringer Kristallisation zur Folge.

Als lichtempfindliche Emulsionen eignen sich Emulsionen von Silberhalogeniden wie Silberchlorid, Silberbromid oder Gemischen davon, evtl. mit einem geringen Gehalt an Silberiodid bis zu 10 Mol-% in einem der üblicherweise verwendeten hydrophilen Bindemittel wie Protein, insbesondere Gelatine, Polyvinylalkohol, Polyvinylpyrolidon, Cellulosederivaten, wie Carboxyalkylcellulose, insbesondere Carboxymethylcellulose oder Derivaten der Alginsäure.

Die Emulsionen können auch chemisch sensibilisiert werden, z.B. durch Zusatz schwefelhaltiger Verbindungen bei der chemischen Reifung, beispielsweise Allylisothiocyanat, Allylthioharnstoff, Natriumthiosulfat und ähnliche. Als chemische Sensibilisatoren können ferner auch Reduktionsmittel, z.B. die in den belgischen Patentschriften 493 464 oder 568 687 beschriebenen Zinnverbindungen, ferner Polyamide wie Diethylentriamin, oder Aminomethansulfinsäurederivate, z.B. gemäß der belgischen Patentschrift 547 323 verwendet werden.

Geeignet als chemische Sensibilisatoren sind auch Edelmetalle bzw. Edelmetallverbindungen wie Gold, Platin, Palladium, Iridium, Rthenium oder Rhodium. Diese Methode der chemischen Sensibilisierung ist in dem Artikel von R. Koslowsky, Z. Wiss. Phot. *46*, 65—72, (1951) beschrieben.

Es ist ferner möglich, die Emulsionen mit Polyalkylenoxidderivaten zu sensibilisieren, z.B. mit Polyethylenoxid eines Molekulargewichts zwischen 1000 und 20000, ferner mit Kondensationsprodukten von Alkylenoxiden und aliphatischen Alkoholen, Glykolen, cyclischen Dehydratisierungsprodukten von Hexitolen, mit alkyl-substituierten Phenolen, aliphatischen Carbonsäuren, aliphatischen Aminen, aliphatischen Diaminen und Amiden. Die Kondensationsprodukte haben ein Molekulargewicht von mindestens 700, vorzugsweise von mehr als 1000. Zur Erzielung besonderer Effekte kann man diese Sensibilisatoren selbstverständlich kombiniert verwenden, wie in der belgischen Patentschrift 537 278 und in der britischen Patentschrift 727 982 beschrieben.

Die farbkupplerhaltigen Emulsionen können ferner spektrale Sensibilisatoren enthalten, z.B. die üblichen Mono- oder Polymethinfarbstoffe, wie Cyanine, Hemicyanine, Streptocyanine, Merocyanine, Oxonole. Hemioxonole, Styrylfarbstoffe oder andere, auch drei- oder mehrkernige Methinfarbstoffe, beispielsweise Rhodacyanine oder Neocyanine. Derartige Sensibilisatoren sind beispielsweise beschrieben in dem Werk von F. M. Hamer "The Cyanine Dyes and Related Compounds", (1964) Interscience Publishers John Wiley and Sons. Vorzugsweise werden die erfindungsgemäßen Farbkuppler jedoch in solchen Emulsionen verwendet, die für rotes Licht sensibilisiert sind.

Die Emulsionen können die üblichen Stabilisatoren enthalten, wie z.B. homöopolare oder salzartige Verbindungen des Quecksilbers mit aromatischen oder heterocyclischen Ringen, wie Mercaptotriazole, einfache Quecksilbersalze, Sulfoniumquecksilberdoppelsalze und andere Quecksilberverbindungen. Als Stabilisatoren sind ferner geeignet Azaindene, vorzugsweise Tetra- oder Pentaazaindene, insbesondere solche, die mit Hydroxyl- oder Aminogruppe substituiert sind. Derartige Verbindungen sind in dem Artikel von Birr, Z. Wiss. Phot. *47*, 2—27 (1952) beschrieben. Weitere geeignete Stabilisatoren sind u.a. heterocyclische Mercaptoverbindungen, z.B. Phenylmercaptotetrazol, quaternäre Benzthiazolderivate, Benztriazol und ähnliche.

Zur Herstellung einer fotografischen Emulsionschicht können außerdem ein oder mehrere Kuppler der angegebenen Formel auch zusammen mit einem oder mehreren bekannten Kupplern angewendet werden, beispielsweise mit Masken- oder DIR-Kupplern.

Die Emulsionen können in der üblichen Weise gehärtet sein, beispielsweise mit Formaldehyd oder halogensubstituierten Aldehyden, die eine Carboxylgruppe enthalten, wie Mucobromsäure, Diketonen, Methansulfonsäureester, Dialdehyden und dergleichen.

Zur Erzeugung des blaugrünen Teilbildes werden die gebräuchlichen Farbentwickler verwendet, z.B. die üblichen aromatischen, mindestens eine primäre Aminogruppe enthaltende Verbindungen des para-Phenylendiamintyps.

Brauchbare Farbentwickler sind beispielsweise N,N-Dimethyl-p-phenylendiamin, N,N-Diethyl-p-phenylendiamin, Monomethyl-p-phenylendiamin, 2-Amino-5-diethylaminotoluol, N-Butyl-N-ω-sulfobutyl-p-phenylendiamin, 2-Amino-5-(N-ethyl-N-β-methansulfonamidoethyl-amino)-toluol und dergleichen. Weitere brauchbare Farbentwickler sind beispielsweise beschrieben in J. Amer. Chem. Soc. *73*, 3100 bis 3125 (1951).

Anhand der folgenden Versuche werden die Vorteile der erfindungsgemäßen Kuppler demonstriert.

Beispiel 1

Bestimmung der Kupplungskinetik

Die Kupplungskinetik wurde als keff nach der in US—A—4 315 070 und DE—A—2 853 362 beschriebenen Methode bestimmt. Die Kuppler wurden dazu nach der im folgenden beschriebenen allgemeinen Vorschrift emulgiert.

100 g Farbkuppler werden in einem Gemisch aus 80 g Dibutylphthalat und 300 ml Ethylacetat unter Erwärmen auf 50°C gelöst und sodann unter Zuhilfenahme einer hochtourigen Mischsirene des Typs MS 1 (Kotthoff GmbH, Köln) in 1 kg einer auf 50°C erwärmten und mit 50 ml einer 10 %igen Lösung des Na-Salzes der Di-sek.-butylnaphthalinsulfonsäure abgemischten 8 %igen Gelatinelösung eingerührt. Danach wurde der Essigester im Vakuum abgedampft und das Emulgat bei 6°C erstarrt und gelagert.

Aus Tabelle 1 lassen sich die erhaltenen Kinetikwerte ersehen.

Die erfindungsgemäßen Kuppler 1, 4 und 6 haben einen beträchtlich höheren keff-Wert als die Vergleichskuppler A, B und C des Standes der Technik.

Tabelle 1

| Kuppler Nr. | keff $\left[\frac{1}{Mol \cdot sec}\right]$ |
|---|---|
| 1 | 6.000 |
| 4 | 5.700 |
| 6 | 5.800 |
| A | 3.500 |
| B | 890 |
| C | 3.400 |

Kuppler A:

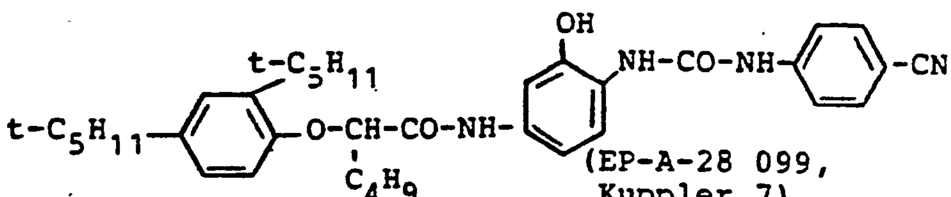

(EP-A-28 099, Kuppler 7)

Kuppler B:

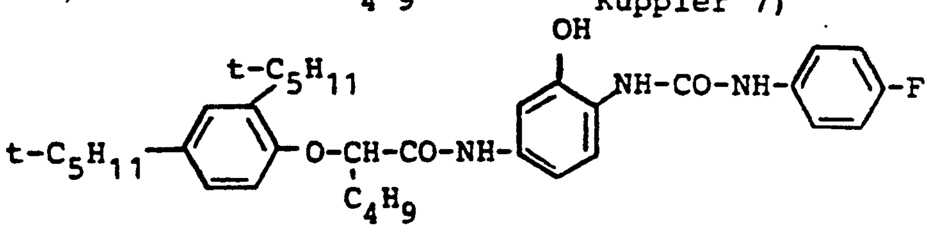

(nach Research Disclosure 24734)

Kuppler C:

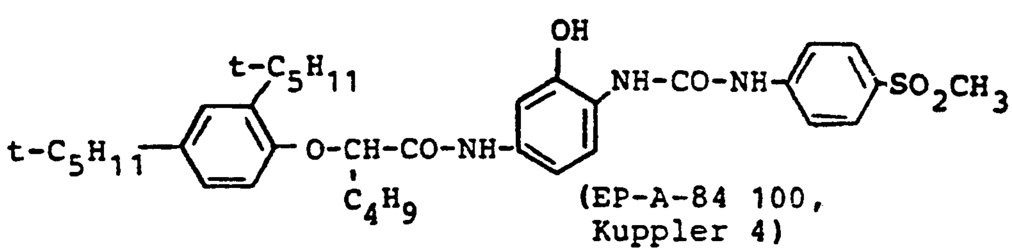

(EP-A-84 100, Kuppler 4)

Beispiel 2

Auf einem mit einer Lichthofschutzschicht versehenen transparenten Schichtträger wurden nacheinander die folgenden Schichten aufgetragen. Die Mengenangaben beziehen sich jeweils auf 1 $m^2$. Für den Silberhalogenidauftrag werden die Mengen als g $AgNO_3/m^2$ angegeben.

1. Eine weniger empfindliche Schicht mit einer rotsensibilisierten Silberbromidiodidemulsion (mit 6 Mol-% $I^{\ominus}$) aus 2,9 $AgNO_3$ und 1,5 g Gelatine mit 0,45 g hierin eingelagertem Blaugrünkuppler.

2. Eine höher empfindliche Schicht mit einer rotsensibilisierten Silberbromidiodidemulsion (mit 8 Mol-% $I^{\ominus}$) aus 3,5 g $AgNO_3$ und 2,2 g Gelatine mit 0,2 g hierin eingelagertem Blaugrünkuppler (die gleiche Verbindung wie in Schicht 1).

3. Eine 0,8 g Gelatine enthaltende Zwischenschicht.

4. Eine Härtungsschutzschicht aus 0,3 g Gelatine, über die 0,4 g eines wasserlöslichen Carbamoylpyridiniumsalzes als Härtungsmittel eingebracht werden.

Die Emulgierung der Blaugrünkuppler erfolgte wie in Beispiel 1 beschrieben.

Jeweils zwei Proben des Materials wurden hinter einem graduierten Graukeil belichtet und gemäß dem bei Ernest Ch. Gehret, British Journal of Photography 1974, S. 597, beschriebenen Verarbeitungsgang verarbeitet. Die 1. Probe wurde unmittelbar nach der Verarbeitung ausgewertet, die 2. jedoch erst nach 4-wöchiger Lagerung bei 80°C und 40% r.F. Gemessen wurden die Absorptionsmaxima bei D = 0,5 und D = 2,0 sowie die Höhe des gb-Schleiers. Aus der Tabelle 2 läbt sich deutlich ersehen, daß der erfindungsgemäße Kuppler im Vergleich zu bekannten Kupplern ein von der Dichte unabhängiges $\lambda_{max}$ hat — das gilt sogar im Vergleich zu einem naphtholischen bg-Kuppler mit der Struktur D — und daß der Vergilbungsgrad des entwickelten Materials geringer ist.

Tabelle 2

| Kuppler Nr. | λ max (frisch) nm D = 0,5 | D = 2,0 | Darkfading-Stabilität nach Lagerung (4 Wochen/80°C/40 % r.F.) Dichteabnahme (%) | Zunahme des gb-Schleiers |
|---|---|---|---|---|
| 4 | 698 | 698 | 4 | 0,13 |
| A | 685 | 687 | 4 | 0,18 |
| C | 681 | 690 | 4 | 0,17 |
| D | 690 | 700 | 57 | 0,14 |
| E | 678 | 684 | 7 | 0,17 |

Kuppler D:

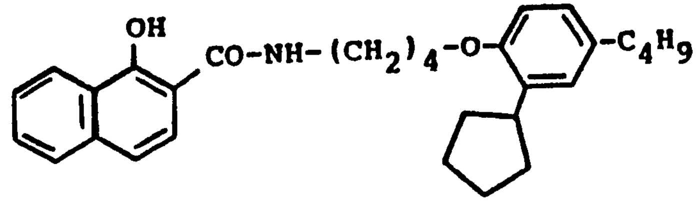

(DE-C-22 14 060, Kuppler 6)

Kuppler E:

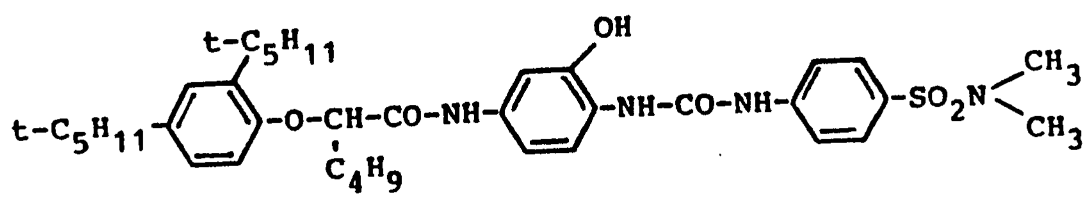

(entspricht EP-A-67 689, Beispiel 81).

**Patentansprüche**

1. Lichtempfindliches farbfotografisches Aufzeichnungsmaterial mit mindestens einer Silberhalogenidemulsionsschicht und einem dieser zugeordneten nichtdiffundierenden phenolischen Blaugrünkuppler mit einer 2-Phenylureidogruppe, dadurch gekennzeichnet, daß der Blaugrünkuppler die folgende Formel hat:

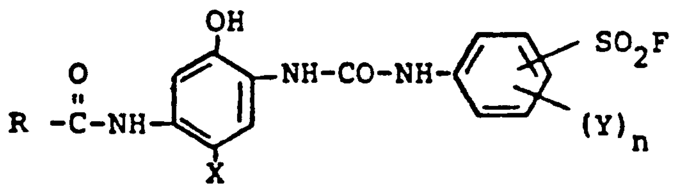

worin bedeuten:
R = Ballastgruppe
X = H oder bei der Farbkupplung abspaltbare Fluchtgruppe
Y = Halogen, Alkyl, Alkoxy, Aryl oder Alkylthio
n = 0—2; falls n = 2, können die Reste Y gleich oder verschieden sein.

2. Lichtempfindliches farbfotografisches Aufzeichnungsmaterial nach Anspruch 1, dadurch gekennzeichnet, daß die Ballastgruppe R die folgende Formel hat:

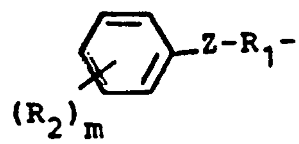

worin bedeuten:
Z = O oder S
$R_1$ = eine Alkylidengruppe mit 2 bis 20 C-Atomen der Formel $R_3$—C-Alkyl, wobei $R_3$ H oder Alkyl bedeutet.
$R_2$ = Hydroxy, Carboxy, Alkyl, Cycloalkyl, Aryl, Aralkyl, Alkoxy, Aryloxy, Alkylsulfamoyl, Arylsulfamoyl, Alkylsulfonamido, Arylsulfonamido, Alkylsulfonyl, Arylsulfonyl, Alkoxycarbonyl oder Acyloxy, worin Alkyl 1 bis 20 C-Atome enthält, worin Aryl 6 bis 20 C-Atome enthält, und worin Alkyl, Aryl und Aralkyl auch beliebig mit Hydroxy, Carboxy, Alkoxycarbonyl oder Acyloxy substituiert sein können,
m = 1—3.

**Revendications**

1. Matériau d'enregistrement photosensible de photographie en couleurs comportant au moins une couche d'émulsion à l'halogénure d'argent et un copulant bleu-vert phénolique attribué à cette couche et ne se diffusant pas, tout en comportant un groupe 2-phényluréido, caractérisé en ce que le copulant bleu-vert répond à la formule suivante:

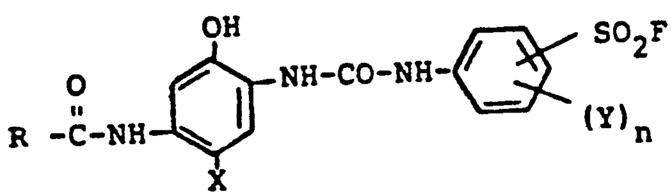

dans laquelle
R = groupe de ballastage
X = H ou un groupe qui s'éloigne et dissociable lors de la copulation chromogène,
Y = halogène, groupe alkyle, groupe alcoxy, groupe aryle ou groupe alkylthio,
n = 0—2; si n = 2, les radicaux Y peuvent être identiques ou différents.

2. Matériau d'enregistrement photosensible de photographie en couleurs selon la revendication 1, caractérisé en ce que le groupe de ballastage R répond à la formule suivante:

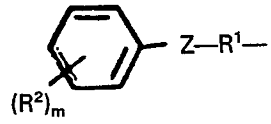

dans laquelle

Z = O ou S,

$R^1$ = un groupe alkylidène contenant 2 à 20 atomes de carbone et répondant à la formule $R^3$—C-alkyle, $R^3$ représentant H ou un groupe alkyle,

$R^2$ = un groupe hydroxyle, un groupe carboxyle, un groupe alkyle, un groupe cycloalkyle, un groupe aryle, un groupe aralkyle, un groupe alcoxy, un groupe aryloxy, un groupe alkylsulfamoyle, un groupe arylsulfamoyle, un groupe alkylsulfonamido, un groupe arylsulfonamido, un groupe alkylsulfonyle, un groupe arylsulfonyle, un groupe alcoxycarbonyle ou un groupe acyloxy, le groupe alkyle contenant 1 à 20 atomes de carbone, tandis que le groupe aryle contient 6 à 20 atomes de carbone et que les groupes alkyle, aryle et aralkyle peuvent également être substitués de manière quelconque par un groupe hydroxyle, un groupe carboxyle, un groupe alcoxycarbonyle ou un groupe acyloxy,

m = 1—3.

**Claims**

1. Light-sensitive colour-photographic recording material having at least one silver halide emulsion layer and a non-diffusing phenolic blue-green coupler with a 2-phenylureido group, characterised in that the blue-green coupler has the following formula:

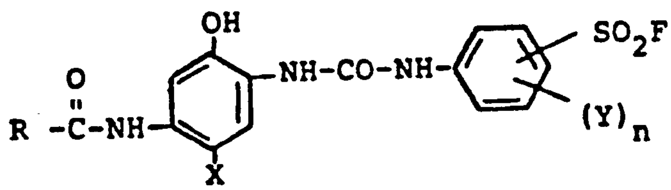

wherein:

R = ballast group

X = H or a labile group which can be eliminated during colour coupling

Y = halogen, alkyl, alkoxy, aryl or alkylthio

n = 0—2; if n = 2, the residues Y can be the same or different.

2. Light sensitive colour-photographic recording material according to claim 1, characterised in that the ballast group R has the following formula:

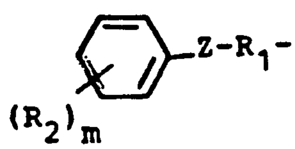

wherein:

Z = O or S

$R_1$ = an alkylidene group having 2 to 20 C atoms with the formula $R_3$—C-alkyl, whereby $R_3$ means H or alkyl,

$R_2$ = hydroxy, carboxyl, alkyl, cycloalkyl, aryl, aralkyl, alkoxy, aryloxy, alkylsulphamoyl, arylsulphamoyl, alkylsulphonamide, arylsulphonamide, alkylsulphonyl, arylsulphonyl, alkoxycarbonyl or acyloxy, in which alkyl has 1 to 20 C atoms, aryl has 6 to 20 C atoms, and alkyl, aryl, and aralkyl can optionally also be substituted with hydroxy, carboxy, alkoxy carbonyl or acyloxy,

m = 1—3.